# EUROPÄISCHE PATENTSCHRIFT

(11) **EP 3 383 455 B1**
(45) Veröffentlichungstag und Bekanntmachung des Hinweises auf die Patenterteilung: **27.04.2022**
(21) Anmeldenummer: 16805363.5
(22) Anmeldetag: 29.11.2016
(51) Int. Cl.: A61M 5/14

(54) **MEDIZINISCHER FLUIDBEUTELHALTER UND VERFAHREN ZU SEINER HERSTELLUNG, MEDIZINISCHE WIEGEVORRICHTUNG UND BEHANDLUNGSVORRICHTUNG**
MEDICAL FLUID BAG HOLDER AND METHOD FOR PRODUCING SAME, MEDICAL WEIGHING DEVICE AND TREATMENT DEVICE
SUPPORT POUR POCHE DE FLUIDE MÉDICAL ET SON PROCÉDÉ DE FABRICATION, DISPOSITIF DE PESAGE MÉDICAL ET DISPOSITIF DE TRAITEMENT

(30) Priorität: 03.12.2015 DE 102015121065; 12.02.2016 DE 102016102498
(43) Veröffentlichungstag der Anmeldung: 10.10.2018
(73) Patentinhaber: Fresenius Medical Care Deutschland GmbH, 61352 Bad Homburg (DE)
(72) Erfinder: MAGER, Gerhard, 61352 Bad Homburg (DE); OESTERREICH, Stefan, 61267 Neu-Anspach (DE); WÄBER, Udo, 63071 Offenbach (DE)
(74) Vertreter: Bobbert & Partner Patentanwälte PartmbB
(86) Internationale Anmeldenummer: PCT/EP2016/079097
(87) Internationale Veröffentlichungsnummer: WO 2017/093235

(56) Entgegenhaltungen:
- DE-A1-102013 000 454
- DE-U1- 20 018 130
- DE-U1- 29 902 375
- JP-A- H02 182 262
- US-A1- 2013 168 526

## Beschreibung

Die vorliegende Erfindung betrifft einen medizinischen Fluidbeutelhalter gemäß Anspruch 1. Sie betrifft zudem ein Verfahren gemäß Anspruch 6, eine medizinische Wiegevorrichtung gemäß Anspruch 7 und eine medizinische Behandlungsvorrichtung gemäß Anspruch 15.

Aus dem Stand der Technik sind Fluidbeutelhalter zum Halten von Beuteln mit medizinischen Fluiden bekannt, siehe beispielsweise JP H02 182262 A.

Eine Aufgabe der vorliegenden Erfindung ist es, einen weiteren Fluidbeutelhalter vorzuschlagen. Zudem sollen ein geeignetes Verfahren zu seiner Herstellung, eine Wiegevorrichtung mit einem derartigen Fluidbeutelhalter und eine Behandlungsvorrichtung angegeben werden.

Die erfindungsgemäße Aufgabe wird durch einen medizinischen Fluidbeutelhalter mit den Merkmalen des Anspruchs 1 gelöst. Sie wird ferner gelöst mittels des Verfahrens mit den Merkmalen des Anspruchs 6, mittels der medizinischen Wiegevorrichtung mit den Merkmalen des Anspruchs 7 und mittels der medizinischen Behandlungsvorrichtung mit den Merkmalen des Anspruchs 15.

Erfindungsgemäß wird somit ein medizinischer Fluidbeutelhalter vorgeschlagen, welcher zum Anhängen von Beuteln mit medizinischem Fluid hieran dient. Der Fluidbeutelhalter weist wenigstens einen Bodenabschnitt auf. Er weist ferner wenigstens zwei Stützarme auf, welche sich vom wenigstens einen Bodenabschnitt erstrecken. Schließlich weist er wenigstens einen Beutelhaltearm auf, welcher sich - mittelbar oder unmittelbar - vom wenigstens einen Bodenabschnitt oder von einem der Stützarme erstreckt.

Das erfindungsgemäße Verfahren dient dem Herstellen eines erfindungsgemäßen Fluidbeutelhalters, insbesondere in dessen einfachster Ausgestaltung gemäß Anspruch 1 oder eines Grundkörpers hiervon bestehend aus einem Bodenabschnitt, zwei Stützarmen und wenigstens einem Beutelhaltearm mit oder ohne Haken. Das Verfahren weist zumindest folgende Schritte auf:
- Stanzen oder Laserschneiden von einem, zwei oder mehreren Blechstreifen oder Rohlingen unter Erzeugen eines Bodenabschnitts, wenigstens zweier Stützarme, welche sich vom Bodenabschnitt - mittelbar oder unmittelbar - erstrecken, und wenigstens eines Beutelhaltearms, wobei sich Bodenabschnitt, Stützarme und Beutelhaltearme auf einem oder zwei Blechstreifen befinden;
- Biegen und/oder Kanten des oder der Blechstreifen; und
- Verbinden des einen Blechstreifens mit sich selbst oder der zwei oder mehr Blechstreifen miteinander derart, dass eine geschlossene oder umlaufende Struktur entsteht.

Die Erfindung betrifft ferner eine medizinische Wiegevorrichtung mit wenigstens je einem erfindungsgemäßen Fluidbeutelhalter, einer Wiegeeinrichtung und einer hiermit vorzugsweise lösbar verbundenen Waagschale.

Die Waagschale weist wenigstens eine Seitenfläche oder einen anderen Abschnitt auf mit einer einem Inneren oder Zentrum der Waagschale zugewandten Erhebung. Die Erhebung dient der formschlüssigen, lösbaren Verbindung zwischen Waagschale und Stützarm, indem die Erhebung vorzugsweise im Spalt oder in der Durchgangsöffnung des Stützarms des Fluidbeutelhalters aufgenommen wird.

Schließlich betrifft die Erfindung eine medizinische Behandlungsvorrichtung, welche wenigstens einen erfindungsgemäßen Fluidbeutelhalter oder wenigstens eine erfindungsgemäße Wiegevorrichtung aufweist.

Bei allen Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Erfindungsgemäße Ausführungsformen können eines oder mehrere der vorstehend oder im Folgenden genannten Merkmale aufweisen. Erfindungsgemäße Ausführungsformen sind ferner Gegenstand der Unteransprüche.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs und/oder die Darstellung in den hier beigefügten Figuren beziehen.

Die vorliegende Erfindung betrifft jede beliebige Kombination von hierin genannten Merkmalen, sofern eine konkrete Kombination nicht für den Fachmann erkennbar technisch unmöglich ist.

In gewissen beispielhaften, erfindungsgemäßen Ausführungsformen sind der Bodenabschnitt und die wenigstens zwei Stützarme einstückig oder integral. Vorzugsweise sind sie aus einem gemeinsamen ersten Materialstreifen, insbesondere Blechstreifen, gefertigt. Dies spart Herstellungsschritte ein.

In manchen beispielhaften, erfindungsgemäßen Ausführungsformen sind der Bodenabschnitt und der wenigstens eine Beutelhaltearm einstückig oder integral, vorzugsweise aus einem gemeinsamen, ersten Materialstreifen, insbesondere Blechstreifen, gefertigt.

In bestimmten beispielhaften, erfindungsgemäßen Ausführungsformen sind wenigstens einer der Stützarme und der wenigstens eine Beutelhaltearm einstückig oder integral, vorzugsweise aus einem gemeinsamen, ersten Materialstreifen, insbesondere Blechstreifen, gefertigt.

In manchen beispielhaften, erfindungsgemäßen Ausführungsformen weist der Fluidbeutelhalter einen oder genau einen Beutelhaltearm auf, welcher einen zylindrischen Querschnitt hat, eine Säule ist oder einen Säulenabschnitt hat und/oder direkt oder indirekt mit dem Bodenabschnitt, vorzugsweise mit einem mittleren Abschnitt hiervon, verbunden ist.

In bestimmten beispielhaften, erfindungsgemäßen Ausführungsformen weist der Fluidbeutelhalter wenigstens zwei Beutelhaltearme auf, welche an jeweils einander gegenüber liegenden Seiten, insbesondere Querseiten, des Bodenabschnitts mit dem Bodenabschnitt verbunden sind oder einstückig oder integral aus diesem hervorgehen.

Zwei der Stützarme sind an jeweils einander gegenüber liegenden Seiten des Bodenabschnitts mit diesem verbunden oder gehen einstückig oder integral aus diesem hervor.

In manchen beispielhaften, erfindungsgemäßen Ausführungsformen weist der Fluidbeutelhalter wenigstens zwei Beutelhaltearme auf, welche an jeweils einander gegenüber liegenden Seiten des Bodenabschnitts mit diesem verbunden sind oder einstückig oder integral aus diesem hervorgehen. In gewissen, beispielhaften erfindungsgemäßen Ausführungsformen sind dies nicht jene Seiten, an welchen die Stützarme mit dem Bodenabschnitt verbunden sind.

In gewissen beispielhaften, erfindungsgemäßen Ausführungsformen weist der Fluidbeutelhalter wenigstens zwei Beutelhaltearme auf, welche mittels wenigstens einer Verbindungsstrebe oder eines Verbindungsabschnitts miteinander verbunden sind, insbesondere in einem oberen Endbereich der Beutelhaltearme.

In gewissen beispielhaften, erfindungsgemäßen Ausführungsformen ist die wenigstens eine Verbindungsstrebe ebenfalls ein Materialstreifen, z. B. Blechstreifen, insbesondere ein integraler Abschnitt des ersten Materialstreifens oder Blechstreifens.

In einigen beispielhaften, erfindungsgemäßen Ausführungsformen weist wenigstens einer der Stützarme wenigstens zwei Seitenstreben auf, welche durch einen offenen Spalt oder eine umschlossene Durchgangsöffnung voneinander beabstandet sind.

In manchen beispielhaften, erfindungsgemäßen Ausführungsformen sind die Seitenstreben ebenfalls Blechstreben oder Blechstreifen, vorzugsweise Abschnitte des ersten Blechstreifens.

In gewissen, beispielhaften erfindungsgemäßen Ausführungsformen weisen wenigstens eine oder zwei der Seitenstreben - z. B. in ihrem Längsschnitt - wenigstens einen gekrümmten oder gebogenen Abschnitt, einen ersten geraden Abschnitt und einen zweiten geraden Abschnitt auf oder bestehen aus diesen. Der gebogene Abschnitt steht näher am Bodenabschnitt als der erste gerade Abschnitt, welcher zwischen dem gebogenen Abschnitt und dem zweiten geraden Abschnitt liegt. Der zweite gerade Abschnitt hat einen größeren radialen Abstand zum Bodenabschnitt oder einen größeren Abstand zum Bodenabschnitt als der erste gerade Abschnitt. Diese Ausgestaltung bewirkt, dass sich die Seitenstrebe mit einem geraden Anteil hiervon nach außen neigt, was einer Verklemmung des Fluidbeutelhalters oder auch nur seiner formschlüssigen Aufnahme in einer Waagschale dient.

In manchen beispielhaften, erfindungsgemäßen, beispielhaften Ausführungsformen stehen die Seitenstreben unter einem Winkel zum Bodenabschnitt oder zur Haupterstreckungsrichtung der Beutelhaltearme, welcher ungleich 90° ist.

In gewissen beispielhaften, erfindungsgemäßen Ausführungsformen sind die Seitenstreben nach außen geneigt.

In bestimmten beispielhaften, erfindungsgemäßen Ausführungsformen weist der Bodenabschnitt einen flächigen Abschnitt, vorzugsweise aus Blech, auf oder besteht hieraus.

In manchen beispielhaften, erfindungsgemäßen Ausführungsformen weist der flächige Abschnitt, vorzugsweise in einem mittleren Abschnitt hiervon, eine Durchgangsöffnung oder eine Vertiefung oder Erhöhung, etwa im Sinne einer Ausbeulung, auf.

In gewissen beispielhaften, erfindungsgemäßen Ausführungsformen sind der erste und/oder der zweite Blechstreifen aus, vorzugsweise rostfreiem, Edelstahl.

In gewissen beispielhaften, erfindungsgemäßen Ausführungsformen haben der erste Materialstreifen und/oder der zweite Materialstreifen, welche aus Blech sein können, eine, vorzugsweise konstante, Dicke, welche vorzugsweise zwischen 1 mm und 8 mm liegt, bevorzugt zwischen 2 mm und 5mm, besonders bevorzugt zwischen 3 mm bis 4 mm.

Der wenigstens eine Beutelhaltearm weist einen oder mehrere Haken zum Anhängen eines oder mehrerer Fluidbeutel auf.

Der oder die Haken sind optional einstückig oder integral mit dem jeweiligen Beutelhaltearm.

In gewissen beispielhaften, erfindungsgemäßen Ausführungsformen weist der Fluidbeutelhalter wenigstens eine Halterung zum lösbaren Halten von Fluidschlauchabschnitten auf.

In bestimmten beispielhaften, erfindungsgemäßen Ausführungsformen weist der Fluidbeutelhalter eine geschlossene oder umlaufende Struktur auf, welche durch das Verbinden des Bodenabschnitts, ein oder zwei Beutelhaltearmen und optional einer Verbindungsstrebe miteinander erzeugt wurde. Die Struktur kann den Bodenabschnitt, den oder die Beutelhaltearme und die optionale Verbindungsstrebe umfassen oder hieraus bestehen.

In bestimmten beispielhaften, erfindungsgemäßen Ausführungsformen dient der Bodenabschnitt zum Aufstellen des Fluidbeutelhalters, in anderen nicht.

Der Bodenabschnitt kann eine Aufstellfläche oder -ebene definieren, haben oder sein, oder nicht.

Der Bodenabschnitt kann eine Bodenfläche sein.

Der Bodenabschnitt kann aus Blech sein.

Der Bodenabschnitt kann eine Standfläche sein.

Der Bodenabschnitt kann eine Rahmenstruktur sein oder hieraus bestehen.

Der Bodenabschnitt kann Füße haben.

In manchen, beispielhaften, erfindungsgemäßen Ausführungsformen ist der Fluidbeutelhalter ausgestaltet, um selbständig stehen zu können. Er kann ein Fluidbeutelständer sein.

In gewissen, beispielhaften, erfindungsgemäßen Ausführungsformen sind die Haken für die Beutel aus Blech, etwa entstanden durch einen entsprechenden Zuschnitt des entsprechenden Blechstreifens.

Es können mehr als vier Haken vorgesehen sein.

Wo immer hierin die Rede ist von einem Blechstreifen, so gilt das zum Blechstreifen Gesagte auch für ein Stück Blech, welche keine Streifenform hat oder hatte. Ein Austauschen von "Blechstreifen" gegen "Blechstück" liegt somit ebenfalls im Umfang dieser Erfindung.

In bestimmten beispielhaften, erfindungsgemäßen Ausführungsformen sind die Blechstreifen aus - vorzugsweise gebeiztem und/oder geschliffenem - rostfreiem Edelstahl, z. B. aus V2A oder V4A.

In bestimmten beispielhaften, erfindungsgemäßen Ausführungsformen werden oder sind die Blechstreifen miteinander verschweißt.

Vorzugsweise werden die Blechstreifen ausschließlich mittels gerader Schweißnähte verschweißt.

In bestimmten beispielhaften, erfindungsgemäßen Ausführungsformen werden oder sind die Blechstreifen entgratet.

In bestimmten beispielhaften, erfindungsgemäßen Ausführungsformen werden oder sind die Blechstreifen poliert.

In bestimmten beispielhaften, erfindungsgemäßen Ausführungsformen ist das Material beständig gegen korrosive Medien, insbesondere gegen medizinische Desinfektionsmittel, chlorhaltige Medien, etc.

In bestimmten beispielhaften, erfindungsgemäßen Ausführungsformen sind Fluidbeutelhalter und Waagschale derart aufeinander abgestimmt, dass die Seitenstreben der Stützarme beim Einsetzen des Fluidbeutelhalters in die Waagschale auf wenigstens einem Abschnitt der Seitenstreben an Seitenflächen oder Stirnseiten der Erhebung oder Erhebungen der Waagschale angeordnet und/oder hiermit in Kontakt sind.

In bestimmten beispielhaften, erfindungsgemäßen Ausführungsformen der Wiegevorrichtung ist der wenigstens eine Abschnitt, in welchem die Seitenstreben mit wenigstens einem Abschnitt hiervon an Seitenflächen oder Stirnseiten der Erhebung in Kontakt kommen, Teil jenes Endabschnitts der Seitenstreben, welcher dem Bodenabschnitt abgewandt oder bodenabschnitt-fern ist.

In bestimmten beispielhaften, erfindungsgemäßen Ausführungsformen sind Fluidbeutelhalter und Waagschale derart aufeinander abgestimmt, dass Querstreben der Stützarme beim Einsetzen des Fluidbeutelhalters in die Waagschale nicht auf oberen Flächen oder Stirnseiten der Erhebung oder Erhebungen der Waagschale zum Liegen kommen.

In gewissen erfindungsgemäßen, beispielhaften Ausführungsformen stehen die Querstreben von einander gegenüber liegenden Stützarmen weiter auseinander als andere, z. B. mittlere, Abschnitte der Seitenstreben.

In bestimmten beispielhaften, erfindungsgemäßen Ausführungsformen stehen die Seitenstreben des einen Stützarms unter einem ersten Winkel zu den Seitenstreben des anderen Stützarms, und die Seitenflächen der Erhebung oder der Erhebungen unter einem zweiten Winkel zueinander. Dabei sind der erste Winkel und der zweite Winkel gleich oder im Wesentlichen gleich.

In bestimmten beispielhaften, erfindungsgemäßen Ausführungsformen sind Fluidbeutelhalter und Waagschale derart aufeinander abgestimmt, dass die Querstreben der Stützarme nach Einsetzen des Fluidbeutelhalters in die Waagschale parallel oder im Wesentlichen parallel zu den oberen Flächen oder Stirnseiten der Erhebung oder Erhebungen der Waagschale stehen.

In bestimmten beispielhaften, erfindungsgemäßen Ausführungsformen weist die Waagschale eine Grundfläche auf, insbesondere eine solche, von welcher sich die Seitenflächen erstrecken, wobei die Grundfläche eine Länge aufweist, welche größer ist als die Länge des Bodenabschnitts oder größer als der Abstand zwischen einem bodenseitigen Abschnitt eines Beutelhaltearms und einem bodenabschnitt-nahen Abschnitt eines diesem Beutelhaltearm gegenüberliegenden weiteren Beutelhaltearms.

In bestimmten beispielhaften, erfindungsgemäßen Ausführungsformen weist die Waagschale an ihren Längsseiten Seitenflächen auf, nicht jedoch an ihren Querseiten. Das Innere des Fluidbeutelhalters ist somit nicht an allen vier Seiten durch senkrechte Strukturen wie Seitenwände oder andere Strukturen mit auch senkrechter Erstreckung begrenzt.

In bestimmten beispielhaften, erfindungsgemäßen Ausführungsformen weist die Erhebung wenigstens ein Maß, vorzugsweise ein Außenmaß, auf, das, vorzugsweise in etwa, einem Innenmaß des Spalts oder der Durchgangsöffnung entspricht.

In manchen beispielhaften, erfindungsgemäßen Ausführungsformen weist die medizinische Behandlungsvorrichtung wenigstens ein Halteblech auf zum Eingreifen in die Rückseite der Erhebung der Waagschale der Wiegevorrichtung.

In gewissen beispielhaften, erfindungsgemäßen Ausführungsformen ist die medizinische Behandlungsvorrichtung als Blutbehandlungsvorrichtung ausgestaltet.

In manchen beispielhaften, erfindungsgemäßen Ausführungsformen ist die medizinische Behandlungsvorrichtung als Akut-Dialysevorrichtung, Dialysevorrichtung, Hämodialysevorrichtung, Hämofiltrationsvorrichtung, Apharesevorrichtung, Plasmabehandlungs- oder Austauschvorrichtung, TPE-(Therapeutic Plasma Exchange)-Vorrichtung, und/oder Kombinationen hiervon ausgestaltet.

In manchen beispielhaften, erfindungsgemäßen Ausführungsformen weist der Fluidbeutelhalter - insbesondere an dem wenigstens einen Bodenabschnitt - keine Rollen, insbesondere keine Rollen eines Rollenwagens als verfahrbarer Ständer zur Aufnahme von Infusionsgeräten, auf.

In manchen beispielhaften, erfindungsgemäßen Ausführungsformen ist der Fluidbeutelhalter zum Einsetzen in eine Waagschale, insbesondere zum lösbaren Einsetzen in eine Waagschale, vorgesehen und ausgestaltet.

In manchen beispielhaften, erfindungsgemäßen Ausführungsformen ist der Fluidbeutelhalter kein verfahrbarer Ständer zur Aufnahme von Infusionsgeräten.

In manchen beispielhaften, erfindungsgemäßen Ausführungsformen ist der Fluidbeutelhalter mit keiner Druckanordnung zum Entleeren eines Fluidbeutels, welcher in oder an dem Fluidbeutelhalter angeordnet ist, verbunden.

In manchen beispielhaften, erfindungsgemäßen Ausführungsformen weist der Fluidbeutelhalter keine Alarmeinrichtung auf, oder ist mit einer solchen verbunden, wobei die Alarmeinrichtung zum Auslösen eines Alarmsignals vorgesehen ist, wenn ein, beispielsweise vorbestimmtes, Gewicht eines Fluidbeutels in dem Fluidbeutelhalter unter einen Alarmwert sinkt.

Einige oder alle erfindungsgemäßen Ausführungsformen können einen oder mehrere der oben oder im Folgenden genannten Vorteile aufweisen.

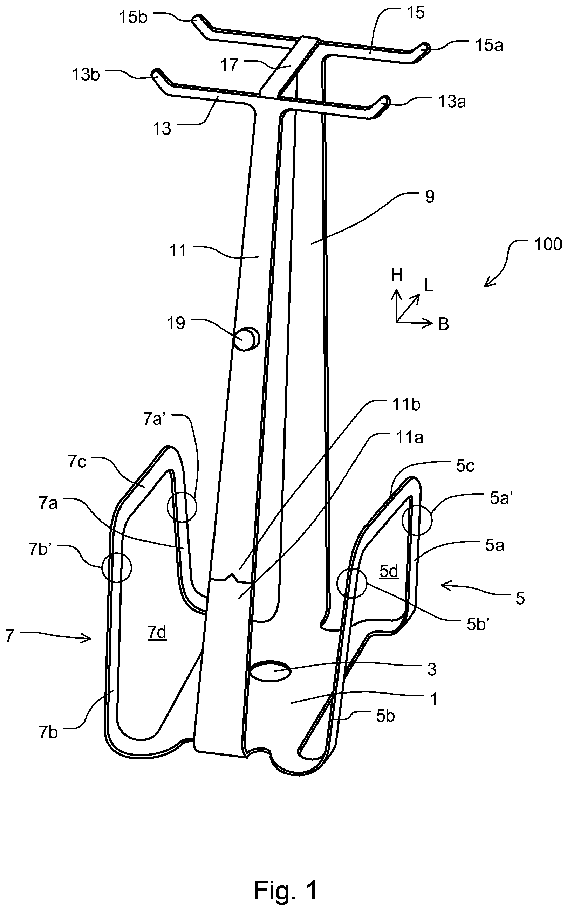

Der erfindungsgemäße Fluidbeutelhalter ist ausgestaltet, um sich mit oberen Abschnitten der Seitenstreben seiner Stützarme an Seitenflächen der Waagschale einer Wiegevorrichtung abzustützen. Da es sich hier um obere Abschnitte handelt, die sich aufgrund ihres Hebelarms wirkungsvoll gegen Erhebungen der Waagschale abstützen und dabei einer Drehung oder einem Verkippen des Fluidbeutelhalters um dessen Breitseite oder um die B-Achse aus Fig. 1 vorbeugen, sorgt dies für einen stabilen Stand des Fluidbeutelhalters in der Waagschale.

Da der Fluidbeutelhalter wie vorstehend ausgeführt aufgrund seines durch die oberen Abschnitte der Seitenstreben der Stützarme und die Erhebungen der Seitenflächen der Waagschale ausgebildeten Verkippschutzmechanismus stabil in der Waagschale steht, können die Breit- oder Querseiten der Waagschale offen bleiben. "Offen bleiben" ist hier so zu verstehen, dass die Querseiten anders als die Längsseiten nicht durch Wände oder Seitenflächen begrenzt sein müssen, gegen welche sich der Fluidbeutelhalter abstützen kann, um nicht zu kippen. Die offenen Querseiten bieten wiederum den Vorteil, dass Fluidbeutel, welche an die Beutelhaltearme angehängt werden, nicht nur von oben (was unbequem sein kann) angehängt werden können sondern auch von der Seite der "fehlenden" Querseiten-Seitenflächen her (was bequemer ist). Zudem erlauben die "fehlenden" Querseiten-Seitenflächen einen ungehinderten Blick auf die angehängten Fluidbeutel und die darin enthaltene Flüssigkeit von der Seite oder aus der Entfernung auch dann, wenn sich im Fluidbeutel, und insbesondere im Bodenbereich des Fluidbeutels nur noch Reste an Flüssigkeit befinden.

Der erfindungsgemäße Fluidbeutelhalter kann ferner Vorteile gegenüber dem Stand der Technik bieten, welche darauf beruhen, dass die zu seiner Herstellung erforderlichen Fertigungsschritte aufgrund der äußerst einfachen Konstruktion des Fluidbeutelhalters automatisiert werden können.

Deshalb, und auch aufgrund der Auswahl des Materials, kann der erfindungsgemäße Fluidbeutelhalter kostengünstig herstellbar sein.

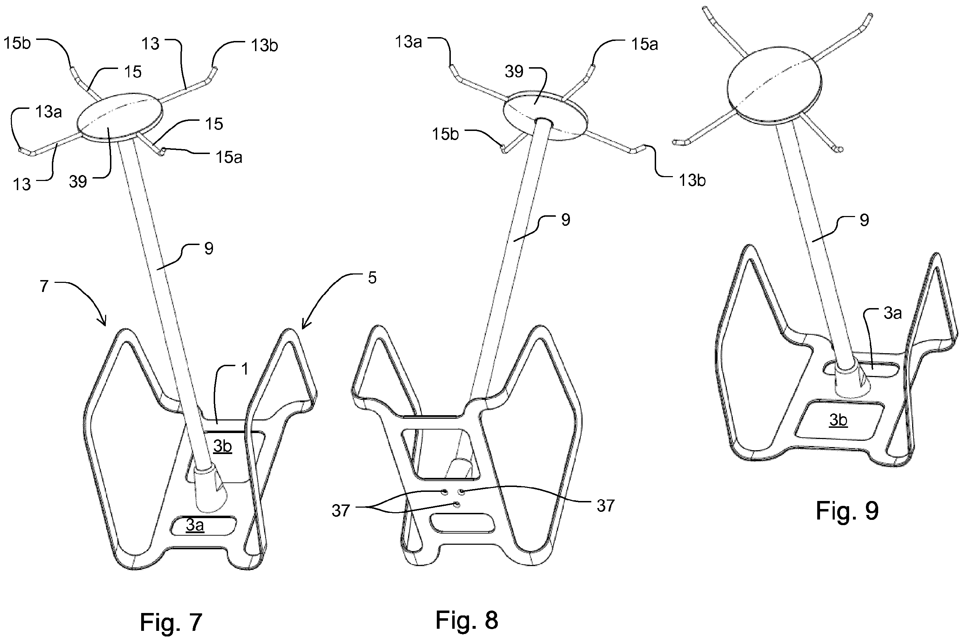

Zugleich kann der erfindungsgemäße Fluidbeutelhalter aufgrund seiner Bauweise und Materialien ein vergleichsweise geringes Gewicht aufweisen. Sein geringes Gewicht kann seine einfachere Handhabung und bessere Wiegeergebnisse erlauben, da niedrigeres Gewicht erlaubt, in einem niedrigeren Messbereich der Waage zu arbeiten wie mit Bezug auf Fig. 7 weiter erläutert ist.

Weitere Vorteile sind insbesondere zu den in den Figuren gezeigten, exemplarischen Ausführungsformen im Folgenden beschrieben.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt einen Fluidbeutelhalter einer ersten erfindungsgemäßen Ausführungsform in perspektivischer Ansicht;
- Fig. 2: zeigt zwei Blechstreifen, aus welchen der Fluidbeutelhalter der Fig. 1 gefertigt werden oder bestehen kann;
- Fig. 3: zeigt in perspektivischer Ansicht den Fluidbeutelhalter der ersten erfindungsgemäßen Ausführungsform, eingesetzt in eine Waagschale;
- Fig. 4: zeigt den Fluidbeutelhalter der Fig. 3, eingesetzt in die Waagschale der Fig. 3, wobei ferner von außen in die Waagschale eingreifende Haltebleche einer Behandlungsvorrichtung dargestellt sind;
- Fig. 5: zeigt einen Fluidbeutelhalter einer zweiten erfindungsgemäßen Ausführungsform in perspektivischer Ansicht;
- Fig. 6: zeigt den Fluidbeutelhalter der zweiten erfindungsgemäßen Ausführungsform in perspektivischer Ansicht von schräg unten;
- Fig. 7: zeigt einen Fluidbeutelhalter einer dritten erfindungsgemäßen Ausführungsform in perspektivischer Ansicht von schräg vorne oben;
- Fig. 8: zeigt den Fluidbeutelhalter der Fig. 7 in perspektivischer Ansicht von schräg unten hinten; und
- Fig. 9: zeigt den Fluidbeutelhalter der Fig. 7 und der Fig. 8 in perspektivischer Ansicht von schräg hinten oben.

**Fig. 1** zeigt den erfindungsgemäßen Fluidbeutelhalter 100 einer ersten, beispielhaften Ausführungsform in perspektivischer Ansicht.

Der Fluidbeutelhalter 100 weist einen Bodenabschnitt 1 mit Längskanten oder -seiten und Querkanten oder -seiten und mit einer optionalen Durchgangsöffnung in einem zentralen oder mittleren Bereich hiervon auf, welche im Folgenden kurz als Öffnung 3 bezeichnet ist. Der Bereich oberhalb der Öffnung 3 gilt hierin als ein Inneres oder als ein innerer Abschnitt des Fluidbeutelhalters 100.

Vom Bodenabschnitt 1 abstehend erheben sich zwei Stützarme 5 und 7, welche von gegenüber liegenden ersten bzw. zweiten Längsseiten des Bodenabschnitts 1 abgehen.

Die Stützarme 5, 7 weisen jeweils zwei seitliche Streben, oder Seitenstreben, 5a und 5b bzw. 7a und 7b auf, welche mittels optionaler Querstreben 5c bzw. 7c optional miteinander verbunden sind. Zu erkennen ist, dass zwischen je zwei Seitenstreben 5a und 5b bzw. 7a und 7b je ein Spalt liegt, welcher aufgrund der optionalen Querstreben 5c, 7c je eine umschlossene Durchgangsfläche 5d bzw. 7d ist. Ohne die optionalen Querstreben 5c, 7c müsste man von einem Spalt, nicht hingegen von einer Durchgangsfläche im Sinne einer Öffnung oder Durchgangsöffnung sprechen.

Vom Bodenabschnitt 1 ausgehend erheben sich ferner zwei Beutelhaltearme 9, 11, welche dem Anhängen von in den Figuren nicht gezeigten, üblichen Fluidbeuteln dienen. Sie gehen ebenfalls aus einander gegenüber liegenden dritten bzw. vierten Querseiten des Bodenabschnitts 1 hervor.

Die Beutelhaltearme 9, 11 können zum Anhängen der vorgenannten Fluidbeutel Haken 13, 15 aufweisen. Diese können sich, z. B. im rechten Winkel, von den Beutelhaltearmen 9, 11 wegstrecken und/oder von diesen abgehen. Sie können die Gestalt von Armen mit gegenüber dem Arm abgewinkelten Spitzen 13a, 13b bzw. 15a, 15b haben.

Die Beutelhaltearme 9, 11 sind in Fig. 1 mittels einer optionalen Verbindungsstrebe 17 miteinander verbunden. Die Verbindungsstrebe 17 kann dabei an jedem Abschnitt der Beutelhaltearme 9, 11 ansetzen. Im Beispiel der Fig. 1 ist dies im Bereich der Haken 13, 15 der Fall, die mittels der Verbindungsstrebe 17 miteinander verbunden sind.

In Fig. 1 sind die Beutelhaltearme 9, 11 wie auch in den folgenden Figuren paarig und als Blechstreifenabschnitte gezeigt. Hiervon abweichend kann aber auch nur ein Beutelhaltearm vorgesehen sein. Ferner können der oder die Beutelhaltearme alternativ auch als Säulen oder Strukturen mit rundem oder elliptischem Querschnitt ausgestaltet sein.

Fig. 1 ist zu entnehmen, dass der Bodenabschnitt 1, die Beutelhaltearme 9, 11 und die Verbindungsstrebe 17 zu einer geschlossenen oder umlaufenden Struktur verbunden sind, welche vielleicht mit einem Rahmen, welcher oben schmaler ist als unten, vergleichbar ist.

Die geschlossene oder umlaufende Struktur ist im Bereich des Bodenabschnitts 1 breiter als im Bereich der Verbindungsstrebe 17, welche zwischen den Beutelhaltearmen 9, 11 angeordnet und mit diesen beiden verbunden ist. Die Verbindungsstrebe 17 kann kürzer sein als der Bodenabschnitt 1 lang ist.

Diese Gestaltung trägt zur höheren Stabilität des Fluidbeutelhalters 100 bei, was wiederum vorteilhaft erlaubt, diesen aus einem einfachen Blechstreifen zu fertigen, der beispielsweise nur mehrfach gebogen und ggf. an einer oder wenigen Stellen mit sich selbst verschweißt oder anderweitig verbunden ist. Ist er verschweißt, so kann die Schweißnaht aufgrund der Streifenform des Blechs gerade verlaufen und vorzugsweise auch noch kurz sein. Dies ist wiederum vorteilhaft, da die Ansprüche an gerade verlaufende Schweißnähte und der Fertigungsaufwand gering sind.

Wenigstens einer der Beutelhaltearme, hier der Beutelhaltearm 11, weist, vorzugsweise an seiner Außenseite, einen oder mehrere Halterungen 19 auf. Diese dienen dem lösbaren Anhängen von Abschnitten von Fluidschläuchen und stellt damit eine gewisse Ordnung der Fluidschläuche sicher. Die Halterungen 19 können auf beliebige Weise mit dem Beutelhaltearm verbunden sein (etwa verschraubt, verschweißt, einstückig oder integral, verklebt, usw.).

Die Beutelhaltearme 9, 11 stehen sich an ihren oberen Enden oder Abschnitten näher als in ihren bodenflächennahen Abschnitten, beispielsweise da die Länge des Bodenabschnitts 1 (in L-Richtung in Fig. 1) bei im Wesentlichen gerade verlaufenden Beutelhaltearmen 9, 11 größer ist als die Länge der Verbindungsstrebe 17. Dies kann vorteilhaft dazu beitragen, dass der an den Haken 13, 15 mit Fluidbeuteln beladene Fluidbeutelhalter 100 ungeachtet des Gewichts der angehängten Beutel stabil stehen kann.

Die Halterungen 19 können im Durchmesser rund sein, sie können zum Verklemmen, zum einfachen Auflegen oder zum Hochhalten des Schlauchabschnitts usw. ausgestaltet sein. Beispielsweise sind sie als Zylinder ausgebildet, die einen Einschnitt in radialer Richtung haben, der vielleicht ein Drittel oder die Hälfte des Durchmessers ausmachen kann, und in welchem die Schlauchabschnitte hineingeklemmt werden können. Sie können jedoch auch als einfache Haken zum Einhängen der Schlauchabschnitte vorgesehen sein.

Fig. 1 ist zu entnehmen, dass der Fluidbeutelhalter 100 dieser exemplarischen Ausführungsform aus einem oder mehr Blechen oder Blechstreifen geformt sein kann, wobei es genügt, einen Blechstreifen zu verwenden und diesen mit sich selbst zum Erzeugen einer geschlossenen Struktur zu verbinden, etwa durch eine einzige, optional auch noch kurze, Schweißnaht.

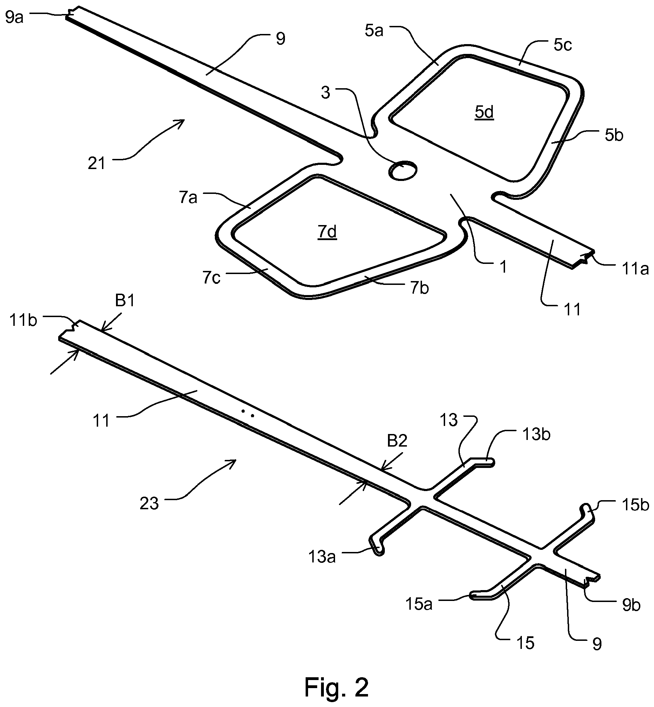

Abweichend von dieser Gestaltung kann der erfindungsgemäße Fluidbeutelhalter 100 natürlich auch aus zwei oder mehr Blechstreifen oder -abschnitten gefertigt werden oder solche aufweisen, die mit entsprechend mehr Verbindungen miteinander verbunden werden. Ein Beispiel, bei welchem zwei Blechstreifen und zwei Verbindungen, hier zwei, optional kurze, Schweißnähte, verwendet werden, ist in Fig. 2 gezeigt.

Fig. 1 zeigt ferner Stoßkanten 11a, 11b. Sie sind mit Bezug auf Fig. 2 nachstehend erläutert.

Die Seitenstreben, 5a und 5b bzw. 7a und 7b weisen je zwei Endbereiche auf, hier als bodenabschnittnah bzw. als bodenabschnittfern bezeichnet. Die mit den Bezugszeichen 5a', 5b', 7a' und 7b' bezeichneten Endbereiche sind die bodenabschnitt-fernen, also jene, welche dem Bodenabschnitt 1 abgewandt sind.

Das in Fig. 1 eingezeichnete Bezugssystem gibt die Richtung der Höhe H, der Länge L und der Breite B des in Fig. 1 gezeigten Fluidbeutelhalters 100 an. Dieses Bezugssystem gilt auch für die folgenden Fig. 2 bis Fig. 4 und alle darin gezeigten Vorrichtungen.

**Fig. 2** zeigt einen flachen ersten Blechstreifen 21 und einen flachen zweiten Blechstreifen 23, aus welchen der Fluidbeutelhalter 100 der Fig. 1 gefertigt werden oder bestehen kann.

Die Blechstreifen 21 und 23 sind in Fig. 2 nach ihrem Ausstanzen aus je einem Blechstreifen gezeigt. Sie sind noch nicht gebogen und noch nicht zum Erzielen der Gestalt des fertigen Fluidbeutelhalters 100 miteinander verbunden.

Abschnitte der Beutelhaltearme 9, 11 liegen, wie in Fig. 2 erkennbar, jeweils zum Teil auf dem ersten Blechstreifen 21 und zum Teil auf dem zweiten Blechstreifen 23 vor. Diese Abschnitte weisen Stoßkanten 9a und 9b bzw. 11a und 11b auf, an welchen die beiden Blechstreifen 21 und 23, nachdem sie gebogen wurden, verbunden werden.

Der erste Blechstreifen 21 weist zwei Stoßkanten 9a und 11a in seinen Abschnitten der Beutelhaltearme 9, 11 auf und endet an seinen Stirnseiten jeweils hiermit.

Der zweite Blechstreifen 23 weist zwei Stoßkanten 9b und 11b in seinen Abschnitten der Beutelhaltearme 9, 11 auf und endet an seinen Stirnseiten jeweils hiermit.

Nach Biegen der beiden Blechstreifen 21 und 23 in ihre Formen, die in Fig. 1 zu sehen sind, wird die Stoßkante 9a mit der Stoßkante 9b verbunden, z. B. verschweißt. Ebenso wird die Stoßkante 11a mit der Stoßkante 11b verbunden, z. B. verschweißt. Das Ergebnis des Verschweißens ist in Fig. 1 zu sehen, wobei dort jedoch der Stoß der Stoßkante 9a mit der Stoßkante 9b nicht eigens dargestellt ist.

Da die beiden Bleche 21 und 23 flache Streifen sind, genügen, um sie miteinander zu verbinden, zwei, vorzugsweise über die Breite des Streifens reichende, vorzugsweise kurze und/oder gerade Schweißnähte, was die Herstellung einfach und kostengünstig macht.

Im Beispiel der Fig. 2 umfasst der erste Blechstreifen 21 die beiden Stützarme 5, 7, den Bodenabschnitt 1 sowie Teile der Beutelhaltearme 9, 11 oder besteht hieraus. Der zweite Blechstreifen 23 umfasst die beiden Haken 13, 15 sowie Teile der Beutelhaltearme 9, 11 oder besteht hieraus.

Wie Fig. 1 und Fig. 2 zu entnehmen ist, laufen beide Beutelhaltearme 9, 11 jeweils in Richtung auf die Haken 13 bzw. 15 zu, wodurch sich die Breite des jeweiligen Blechstreifens 21, 23 verringert. Dies dient dem Einsparen von Material. Die abnehmende Streifenbreite ist exemplarisch mit B1 und B2 in Fig. 2 bezeichnet, wobei B1 > B2 gilt.

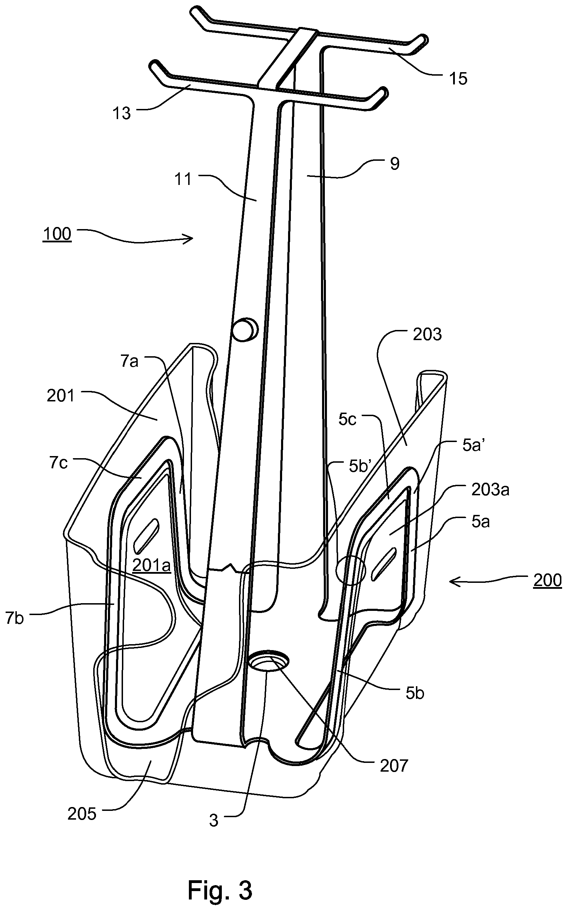

**Fig. 3** zeigt in perspektivischer Ansicht den Fluidbeutelhalter 100 der ersten erfindungsgemäßen Ausführungsform, eingesetzt in eine Waagschale 200.

Die Waagschale 200 ist Teil einer nicht weiter dargestellten Wiegevorrichtung, welche wiederum eine Wiegeeinrichtung aufweist. Die Wiegeeinrichtung ist eingestellt, um das Gewicht des in der Waagschale 200 stehenden Fluidbeutelhalters 100 nicht anzuzeigen. Ihre Gewichtsanzeige steht sozusagen trotz des Eigengewichts des Fluidbeutelhalters 100 auf Null. Wird nun zusätzlich ein Fluidbeutel in die Waagschale 200 gelegt, so zeigt die Wiegeeinrichtung dessen Gewicht an. Wird der Fluidbeutel stattdessen an einen der Haken 13, 15 gehängt, so ändert dies nichts am angezeigten Gewichtswert; die Wiegeeinrichtung zeigt wiederum das Gewicht des Fluidbeutels an.

Die Waagschale 200 weist sich in Fig. 3 nach oben erstreckende Seitenflächen 201 und 203 auf. Diese gehen von einer Grundfläche 205 aus und können, z. B. als Spritzgussteile, einstückig mit dieser sein.

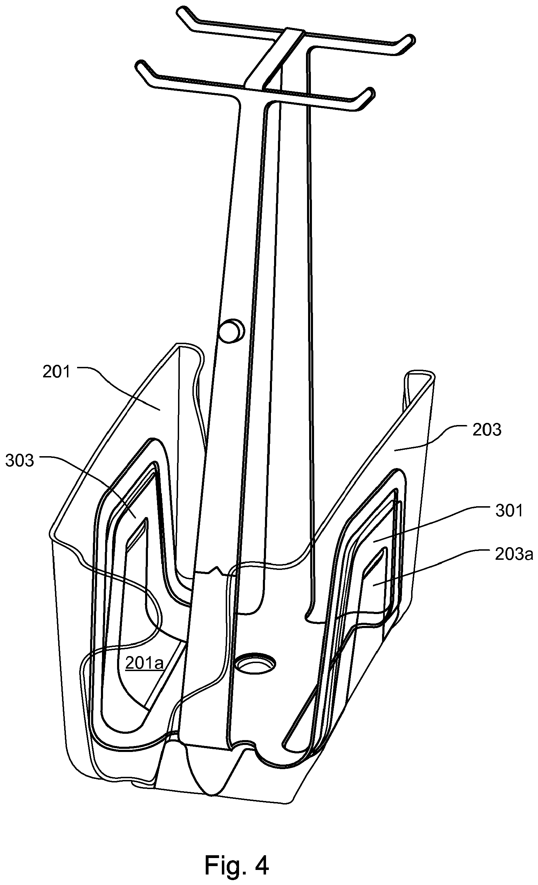

Beide Seitenflächen 201 und 203 weisen in einem mittleren oder zentralen Abschnitt hiervon, jedenfalls in einem Inneren der Waagschale 200, in dem auch der Fluidbeutelhalter 200 steht, je eine Erhebung 201a bzw. 203a auf. Es sind die Vorderseiten der Erhebungen 201a, 203a, welche erhaben sind im Sinne einer Erhöhung gegenüber der übrigen Seitenfläche 201, 203. Die Rückseiten der Erhebungen 201a, 203a können, wie zu Fig. 4 diskutiert wird, optional eine Vertiefung aufzeigen.

Die Erhebungen 201a, 203a haben eine Breite oder Form, welche zumindest dem Abstand zwischen den Seitenstreben 5a, 5b bzw. 7a, 7b, oder der Form des Spalts zwischen den Seitenstreben 5a, 5b bzw. 7a, 7b, oder der Form der Durchgangsöffnung zwischen den Quer- und Seitenstreben 5c, 5a, 5b bzw. 7c, 7a, 7b entsprechen. Auf diese Weise können die Stützarme 5, 7 über jeweils eine der Erhebungen 201a, 203a geschoben und hierdurch ein Formschluss zwischen Spalt oder Durchgangsöffnung 5d, 7d (siehe Fig. 1) einerseits und Erhebung andererseits erzeugt werden. Der Formschluss erlaubt es, ein Kippen des Fluidbeutelhalters 100 um die B-Achse der Fig. 1 zu verhindern, also ein Kippen von vorne nach hinten bezogen auf die Darstellung der Fig. 1.

Verlaufen die Seitenstreben 5a, 5b bzw. 7a, 7b jeweils im Winkel zueinander und vorzugsweise so, dass ihr oberer Abstand größer als ihr unterer Abstand zueinander ist, so dass sich ein sich nach unten weiter öffnender Winkel oder ein sich nach unten öffnendes Trapez ergibt, so erlaubt dies zum einen ein vorteilhaftes Selbstzentrieren und Einnehmen einer Formschlussposition, bei welcher die Seitenstreben 5a, 5b bzw. 7a, 7b jeweils den ebenfalls unter einem Winkel zueinander stehenden seitlichen Begrenzungen der jeweiligen Erhebung 201a, 203a anliegen. Zum anderen erlaubt diese Ausgestaltung ein müheloses Einsetzen des Fluidbeutelhalters 100 in die Waagschale 200, bei dem ein Verklemmen der Seitenstreben 5a, 5b bzw. 7a, 7b an den seitlichen Begrenzungen der jeweiligen Erhebung 201a, 203a vorteilhafter Weise nicht auftritt.

Die Seitenstreben 5a, 5b und 7a, 7b sind optional in einer Richtung der Breite des Fluidbeutelhalters 100 (siehe die B-Richtung in Fig. 1) gebogen, derart, dass sie ausgehend vom Bodenabschnitt 1 oder in einem bodenabschnittnahen Abschnitt einen anderen Winkel relativ zum Bodenabschnitt 1 bilden als jeweils in einem oberen Bereich, bodenabschnittferner Abschnitt oder Spitzenbereich der jeweiligen Seitenstrebe 5a, 5b und 7a, 7b. Dies erlaubt ein einfaches Einsetzen des Fluidbeutelhalters 100 von oben nach unten derart, dass ein durch den Bodenabschnitt 1 gebildeter Abschluss oder eine so gebildete Begrenzung der Durchgangsöffnung 5d, 7d an der Erhebung 201a, 203a vorbei geschoben werden kann.

Die vorstehend beschriebene Ausgestaltung, bei der die Stützarme 5, 7 und die Erhebung 201, 203 wie beschrieben aufeinander abgestimmt sind, erlaubt vorteilhaft ein stabiles, kippfestes Anordnen des Fluidbeutelhalters 100 in der Waagschale 200 unter Formschluss, ohne dass es hierbei eines Befestigungsmechanismus wie etwa eines Klickmechanismus, eines Verschnappmechanismus, eines Verrastmechanismus oder dergleichen bedürfe. Da der Fluidbeutelhalter 100 ohne bewegte Teile an der Waagschale Halt finden kann, wird hiermit eine besonders verschleißfreie und daher vorteilhafte Ausgestaltung vorgeschlagen.

Die Stabilität wird weiter durch die Öffnung 3 erhöht, die bereits aus Fig. 1 hervorgeht. In Fig. 3 ist gezeigt, dass die Öffnung 3 einen Angusshof 207 der hier exemplarisch mittels Spritzgussverfahren hergestellten Waagschale 200 aufnimmt oder umgibt. Auf diese Weise steht der Bodenabschnitt 1 nicht auf dem Angusshof 207; ein aufgrund des Angusshofs 207 wackeliger Stand des Fluidbeutelhalters 100 wird hiermit vorteilhaft verhindert.

Die Öffnung 3 erlaubt somit aber auch, dass mögliche Unebenheiten der Waagschale 200, welche sich durch den (Kunststoff-)Spritzguss ergeben können, verbleiben dürfen, und dass eine entsprechende Nachbearbeitung unterbleiben kann. Auf diese Weise dient die Öffnung 3 dazu, Arbeitsschritte der Nachbearbeitung überflüssig werden zu lassen.

In Fig. 3 ist zu erkennen, dass die Erhebung 201a oder 203a in parallelen Schnitten senkrecht zur Achse H, siehe Fig. 1, unterschiedliche Breiten aufweisen. In derartigen Schnitten weisen auch die Innenseiten der Seitenstreben 5a und 5b bzw. 7a und 7b unterschiedliche Abstände in der Richtung L voneinander auf. Ist der Fluidbeutelhalter 100 in die Waagschale 200 eingesetzt, so entspricht die Breite der Erhebung 201 a und 203a in einem bodenabschnittfernen Schnitt senkrecht zur Achse H dem Abstand der Seitenstreben 5a und 5b oder 7a und 7b voneinander. In anderen, und vor allem in bodenabschnittnäheren Schnitten, ist die Breite der Erhebung jeweils größer als der Abstand zwischen den Seitenstreben, es verbleibt hier ein Spalt zwischen Seitenstreben und Erhebungen. Daher ruht das Gewicht des Fluidbeutelhalters 100 vornehmlich auch in der vorgenannten bodenabschnitt-fernen Schnittebene bzw. dem bodenabschnitt-fernen Abschnitt. Dies trägt zur Hebelarmwirkung und Verkippsicherung bei.

**Fig. 4** zeigt den Fluidbeutelhalter 100 der Fig. 3, eingesetzt in die Waagschale 200 der Fig. 3, wobei ferner in die Waagschale 200 eingreifende Haltebleche 301, 303 einer (im Übrigen nicht gezeigten) Behandlungsvorrichtung dargestellt sind.

Die Haltebleche 301, 303 greifen an einer Rückseite der Seitenflächen 201, 203 der Waagschale 200 in die Rückseite der Erhebungen 201a und 203a ein, die sich in der exemplarischen Ausführungsform der Fig. 4 auf den Rückseiten der Seitenflächen 201 als Vertiefungen darstellen. Die Haltebleche 301, 303 stützen somit die Waagschale 200 und verhindern ihr Verrutschen bezogen auf die Wiegevorrichtung oder die Behandlungsvorrichtung.

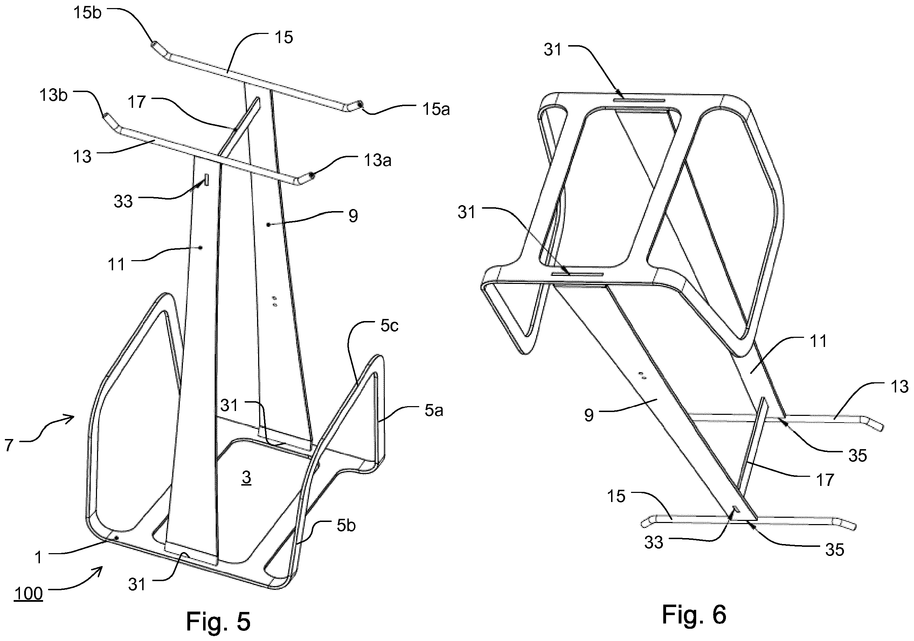

**Fig. 5** zeigt den erfindungsgemäßen Fluidbeutelhalter 100 einer zweiten, beispielhaften Ausführungsform in perspektivischer Ansicht.

Der Fluidbeutelhalter 100 weist in seinem Bodenabschnitt 1 eine im Vergleich mit der ersten Ausführungsform große Öffnung 3 auf. Die Öffnung 3 kann aufgrund ihrer Größe den Bodenabschnitt 1 zur Rahmenstruktur werden lassen, was dazu beitragen kann, Gewicht einzusparen.

Anders als in der ersten Ausführungsform der Fig. 1 sind die Beutelhaltearme 9, 11 jeweils optional einstückig. Sie weisen keine Stoßkanten 9a, 9b bzw. 11a, 11b auf, mittels welcher in der ersten Ausführungsform die beiden Blechstreifen 21 und 23 miteinander verbunden sind.

Die Beutelhaltearme 9, 11 der in Fig. 5 gezeigten zweiten Ausführungsform können wie in Fig. 5 gezeigt exemplarisch in Schlitze oder Nuten 31 eingesteckt sein. Die Schlitze oder Nuten 31 können offen, also durch den Bodenabschnitt 1 von oben nach unten durchgehend sein (Durchgangsöffnungen); sie können alternativ geschlossen sein, also sich nur durch einen Teil der Dicke des Bodenabschnitts 1 erstrecken (Sacklochöffnung).

Die Beutelhaltearme 9, 11 können z. B. passgenau in die Schlitze oder Nuten 31 eingesteckt sein. Sie können zusätzlich oder alternativ zur Passung mittels anderer Fügeverfahren mit dem Bodenabschnitt 1 verbunden sein, etwa verschweißt, verklebt, usw. sein. Die Schlitze oder Nuten 31 können als Schweißhilfe ausgestaltet sein, welche ein gewünschtes Anordnen der Beutelhaltearme 9, 11 am Bodenabschnitt 1 zum Zwecke eines nachfolgenden Schweißens erleichtern. Das Schweißen kann von unten oder von außen erfolgen.

Ebenfalls anders als in der ersten Ausführungsform der Fig. 1 ist die optionale Verbindungsstrebe 17 nicht Teil von Blechstreifen 21 und 23 oder einem der beiden Beutelhaltearme 9, 11. Sie ist im Beispiel der Fig. 5 vielmehr eine eigenständige Komponente, die mit einem oder wie in Fig. 5 gezeigt mit beiden Beutelhaltearmen 9, 11 verbunden ist. Die Verbindungsstrebe 17 kann in Schlitze oder Nuten 33 der Beutelhaltearme 9, 11 eingesteckt sein. Die Schlitze oder Nuten 33 können wie die Schlitze oder Nuten 31 ausgeführt sein. Die Verbindung kann wie vorstehend zur Verbindung zwischen Beutelhaltearmen 9, 11 und Bodenabschnitt 1 ausgeführt sein.

**Fig. 6** zeigt den erfindungsgemäßen Fluidbeutelhalter 100 der Fig. 5 in perspektivischer Ansicht, schräg von unten.

Zu erkennen ist, dass - wiederum anders als in der ersten Ausführungsform der Fig. 1 - die Haken 13, 15 ebenfalls nicht Teil von Blechstreifen oder einem der beiden Beutelhaltearme 9, 11 sind. Sie sind im Beispiel der Fig. 5 und Fig. 6 vielmehr eigenständige Komponenten. Sie können, rein optional, in einem Stoßbereich 35 zwischen Haken 13, 15 und Beutelhaltearmen 9, 11 mittels Schweißnähten mit den Beutelhaltearmen 9, 11 verbunden sein. Andere Fügeverfahren wie Verkleben usw. kommen ebenfalls in Betracht.

**Fig. 7** zeigt den Fluidbeutelhalter einer dritten erfindungsgemäßen Ausführungsform in perspektivischer Ansicht von schräg vorne und schräg oben;

Anders als in der ersten und zweiten Ausführungsform der Fig. 1 bis Fig. 6 weist der Fluidbeutelhalter 100 nur einen Beutelhaltearm 9 auf. Dieser geht optional nicht einstückig aus dem Bodenabschnitt 1 oder einem der beiden Blechstreifen 21 und 23 hervor, sondern ist mit dem Bodenabschnitt 1 verbunden. Im Beispiel der Fig. 7 ist er optional mit einem Mittelbereich des Bodenabschnitts 1 verbunden; andere Bereiche des Bodenabschnitts 1 kommen ebenfalls in Frage. Zur Verbindung stehen wiederum alle bekannten Fügeverfahren zur Verfügung, insbesondere die hierin genannten. Rein exemplarisch ist der Beutelhaltearm 9 mit einem Fuß oder Fußabschnitt hiervon mittels Schrauben 37 (siehe Fig. 8) mit dem Bodenabschnitt 1 verschraubt.

Wie Fig. 7 zeigt, kann der Beutelhaltearm 9 als Stab oder Säule ausgestaltet sein, mit einem beispielsweise runden Querschnitt.

Der Beutelhaltearm 9 steht optional nicht rechtwinklig auf dem Bodenabschnitt 1. Ein Winkel zwischen der Oberseite oder Haupterstreckungsfläche des Bodenabschnitts 1 und der Längsachse oder -erstreckung des Beutelhaltearms 9 nimmt einen Wert zwischen z. B. 65° und 85°, insbesondere zwischen 70 und 80° an. Der nicht rechtwinklig auf dem Bodenabschnitt 1 stehende Beutelhaltearm 9 erlaubt es vorteilhaft, den Beutelhaltearm 9 selbst dann noch - vorzugsweise im Wesentlichen oder vollkommen - vertikal aufzustellen und zu nutzen, wenn der Bodenabschnitt 1 aufgrund von äußeren Rahmenbedingungen (die z. B. auf die Anordnung und Ausrichtung der Waagschale 200 zurückzuführen sind) seinerseits nicht vollkommen horizontal zum Liegen kommt. Dieser Vorteil ergibt sich in besonderem Maße dann, wenn die Stützarme 5, 7 jeweils sowohl rechts als auch links in der Waagschale 200 zum Liegen kommen können, der Fluidbeutelhalter 100 somit also sowohl auf eine erste Weise als auch auf eine zweite, gegenüber der ersten Weise um 180° um eine Achse, die in Höhenrichtung verläuft, gedrehte Weise in die Waagschale 200 eingesetzt werden kann. Letzteres kann optional begünstigt werden durch eine Symmetrie der Stützarme 5, 7.

Der Beutelhaltearm 9 weist an einem freien oder oberen Ende hiervon Haken 13, 15 mit, rein beispielhaft vier, Spitzen oder Endabschnitten 13a, 13b bzw. 15a, 15b auf.

Anders als in der ersten und zweiten Ausführungsform der Fig. 1 bis Fig. 6 treten die Haken 13, 15 aus einer rein exemplarisch vorgesehenen Telleranordnung 39 oder einer anderweitig ausgestalteten Einrichtung aus oder sind mit dieser verbunden. Die Haken 13, 15 treten dabei in mehr als zwei (hier: vier) Richtungen oder Himmelsrichtungen aus, wodurch sie gut zugänglich sind.

Anders als in der ersten und zweiten Ausführungsform der Fig. 1 bis Fig. 6 ist die Öffnung 3 nicht einteilig sondern unterteilt sich in zwei Öffnungen oder Teilöffnungen 3a und 3b. Die Unterteilung erfolgt im Beispiel der Fig. 7 mittels des Mittelbereichs des Bodenabschnitts 1.

Bevorzugt sind die Teilöffnungen 3a, 3b ungleich groß. Im Beispiel der Fig. 7 ist die Teilöffnung 3b ausreichend groß gewählt, um den in Fig. 3 gezeigten Angusshof 207 aufzunehmen oder zu umgeben. Hierdurch ist wiederum sichergestellt, dass der Fluidbeutelhalter 100 trotz des Angusshofs 207 sicheren Stand finden kann. Da der Angusshof 207 in einer Mitte (in Längsrichtung) der Waagschale liegen kann, ist der Beutehalter 9 nicht-mittig (in Längsrichtung) vorgesehen.

Zugleich erlaubt das Vorsehen von zwei Teilöffnungen 3a, 3b (oder auch mehr als nur zwei Teilöffnungen), Material und vor allem Gewicht einzusparen. Dies macht den Fluidbeutelhalter 100 vergleichsweise leichter und damit besser zu handhaben für das Personal, das ihn regelmäßig in Kopfhöhe aufstellen muss. Zudem erlaubt das Einsparen an Gewicht bessere Wiegeergebnisse: der Fluidbeutelhalter 100 steht auf der Waageschale 200, er wird somit mitgewogen. Je geringer sein Gewicht, umso niedriger ist das gewogene Gewicht und in einem umso niedrigeren Messbereich kann die Waage arbeiten. Da Waagen im niedrigeren Messbereich (also bei geringeren Gewichten) i.a.R. genauer wiegen können als in einem höheren Messbereich (also bei größeren Gewichten), trägt das geringe Gewicht des Fluidbeutelhalters 100 nicht nur zu besserer Handhabung, sondern auch zu höherer Messgenauigkeit beim Wiegen vorteilhaft bei.

In der beispielhaften Ausgestaltung der Fig. 7 ist der Beutelhaltearm 9 optional nicht mittig (in Längsrichtung, also von vorne nach hinten, bezogen auf die Darstellung der Fig. 7) angeordnet.

**Fig. 8** zeigt den Fluidbeutelhalter 100 der Fig. 7 in perspektivischer Ansicht von schräg unten und schräg hinten.

**Fig. 9** zeigt den Fluidbeutelhalter 100 der Fig. 7 und der Fig. 8 in perspektivischer Ansicht von schräg hinten und schräg oben.

### Bezugszeichenliste

- 100: Fluidbeutelhalter
- 1: Bodenabschnitt
- 3: Öffnung
- 3a, 3b: (Teil)Öffnungen
- 5: Stützarm
- 5a: Seitenstrebe
- 5a': Endabschnitt
- 5b: Seitenstrebe
- 5b': Endabschnitt
- 5c: Querstrebe
- 5d: Durchgangsfläche oder Durchgangsöffnung
- 7: Stützarm
- 7a: Seitenstrebe
- 7a': Endabschnitt
- 7b: Seitenstrebe
- 7b': Endabschnitt
- 7c: Querstrebe
- 7d: Durchgangsfläche oder Durchgangsöffnung
- 9: Beutelhaltearm
- 9a, 9b: Stoßkanten

- 11: Beutelhaltearm
- 11a, 11b: Stoßkanten
- 13: Haken
- 13a: Spitze oder Endabschnitt
- 13b: Spitze oder Endabschnitt
- 15: Haken
- 15a: Spitze oder Endabschnitt
- 15b: Spitze oder Endabschnitt
- 17: Verbindungsstrebe
- 19: Halterung
- 21: erster Blechstreifen
- 23: zweiter Blechstreifen
- 31: Schlitz oder Nut
- 33: Schlitz oder Nut
- 35: Stoßbereich
- 37: Schrauben
- 39: Telleranordnung

- 200: Waagschale
- 201: Seitenfläche
- 201a: Erhebung der Waagschale
- 203: Seitenfläche
- 203a: Erhebung der Waagschale
- 205: Grundfläche
- 207: Angusshof

- 301: Halteblech
- 303: Halteblech

- B: Breite
- B1: Streifenbreite
- B2: Streifenbreite

- H: Höhe
- L: Länge

## Patentansprüche

1. Medizinischer Fluidbeutelhalter (100), zum Einsetzen in eine Waagschale (200) einer Wiegevorrichtung und zum Anhängen von Beuteln, welche medizinisches Fluid enthalten, wobei der Fluidbeutelhalter (100), der sich jeweils in einer Richtung seiner Höhe (H), einer hierzu senkrechten Richtung seiner Länge (L) und, senkrecht zu den beiden vorstehend genannten Richtungen, einer Richtung seiner Breite (B) erstreckt, aufweist:
- wenigstens einen Bodenabschnitt (1);
- wenigstens einen Beutelhaltearm (9, 11), welcher sich vom Bodenabschnitt (1) erstreckt, wobei der wenigstens eine Beutelhaltearm (9, 11) einen oder mehrere Haken (13, 15) zum Anhängen eines oder mehrerer Fluidbeutel aufweist;
oder hieraus besteht,
**dadurch gekennzeichnet, dass** der Fluidbeutelhalter (100) weiterhin
- wenigstens zwei Stützarme (5, 7) aufweist, welche sich vom Bodenabschnitt (1) erstrecken,
wobei wenigstens zwei der Stützarme (5, 7) an jeweils einander gegenüber liegenden Seiten des Bodenabschnitts (1) mit diesem verbunden sind oder einstückig oder integral aus diesem hervorgehen,
wobei wenigstens einer der Stützarme (5, 7) zwei Seitenstreben (5a, 5b, 7a, 7b) zum Abstützen des Fluidbeutelhalters (1) mittels oberer Abschnitte der Seitenstreben (5a, 5b, 7a, 7b) an sich nach oben erstreckenden Seitenflächen (201, 203) der Waagschale (200) unter Vorbeugen einer Drehung oder einem Verkippen des Fluidbeutelhalters (100) um eine in Richtung seiner Breite (B) verlaufende Achse aufweist, welche durch einen offenen Spalt oder eine umschlossene Durchgangsfläche oder Durchgangsöffnung (5d, 7d) voneinander beabstandet sind.

2. Fluidbeutelhalter (100) nach Anspruch 1, wobei der Bodenabschnitt (1) und die wenigstens zwei Stützarme (5, 7) einstückig, vorzugsweise aus einem gemeinsamen ersten Materialstreifen, insbesondere Blechstreifen (21), gefertigt sind.

3. Fluidbeutelhalter (100) nach einem der vorangegangenen Ansprüche, wobei der Bodenabschnitt (1) und der wenigstens eine Beutelhaltearm (9, 11) einstückig, vorzugsweise aus einem gemeinsamen, ersten Materialstreifen, insbesondere Blechstreifen (21), gefertigt sind.

4. Fluidbeutelhalter (100) nach einem der vorangegangenen Ansprüche, wobei einer der Stützarme (5, 7) und der wenigstens eine Beutelhaltearm (9, 11) einstückig, vorzugsweise aus einem gemeinsamen, ersten Materialstreifen, insbesondere Blechstreifen (21), gefertigt sind.

5. Fluidbeutelhalter (100) nach einem der vorangegangenen Ansprüche, welcher wenigstens zwei Beutelhaltearme (9, 11) aufweist, welche mittels eines Verbindungsabschnitts (17), vorzugsweise einstückig, miteinander verbunden sind.

6. Verfahren zum Herstellen eines Fluidbeutelhalters (100) nach einem der vorangegangenen Ansprüche, welches die folgenden Schritte umfasst oder hieraus besteht:
- Stanzen oder Laserschneiden von einem, zwei oder mehreren Blechstreifen oder Rohlingen unter Erzeugen eines Bodenabschnitts (1), wenigstens zweier Stützarme (5, 7), welche sich vom Bodenabschnitt (1) erstrecken, und wenigstens eines Beutelhaltearms (9, 11), wobei sich Bodenabschnitt (1), Stützarme (5, 7) und Beutelhaltearm (9, 11) auf einem oder zwei Blechstreifen (21, 23) befinden;
- Biegen und/oder Kanten des oder der Blechstreifen (21, 23); und
- Verbinden des einen Blechstreifens (21) mit sich selbst oder der zwei oder mehr Blechstreifen (21, 23) miteinander derart, dass eine geschlossene oder umlaufende Struktur entsteht, wobei der Schritt des Verbindens ein Schweißschritt ist oder einen solchen umfasst.

7. Medizinische Wiegevorrichtung mit wenigstens je:
- einem Fluidbeutelhalter (100) nach einem der Ansprüche 1 bis 5;
- einer Wiegeeinrichtung;
- einer Waagschale (200), verbunden mit einer Wiegeeinrichtung, wobei die Waageschale (200) wenigstens eine Seitenfläche (201, 203) oder einen anderen Abschnitt aufweist mit wenigstens einer Erhebung (201a, 203a), wobei die Erhebung (201a, 203a) geeignet und/oder dimensioniert ist zu ihrer Aufnahme in den Spalt oder in die Durchgangsöffnung (5d, 7d) des Stützarms (5, 7) des Fluidbeutelhalters (100).

8. Wiegevorrichtung nach Anspruch 7, wobei die Stützarme (5, 7) Seitenstreben (5a, 5b, 7a, 7b) aufweisen, welche bei erfolgtem Einsetzen des Fluidbeutelhalters (100) in die Waagschale (200) mit wenigstens einem Abschnitt der Seitenstreben (5a, 5b, 7a, 7b) an Seitenflächen oder Stirnseiten der Erhebung (201a, 203a) zum Liegen kommen.

9. Wiegevorrichtung nach Anspruch 8, wobei der wenigstens eine Abschnitt, in welchem die Seitenstreben (5a, 5b, 7a, 7b) mit wenigstens einem Abschnitt der Seitenstreben (5a, 5b, 7a, 7b) an Seitenflächen oder Stirnseiten der Erhebung (201a, 203a) in Kontakt kommen, Teil jenes
Endabschnitts (5a', 5b', 7a', 7b') der Seitenstreben (5a, 5b, 7a, 7b) ist, welcher dem Bodenabschnitt (1) abgewandt ist.

10. Wiegevorrichtung nach einem der Ansprüche 7 bis 9, wobei die Stützarme (5, 7) Querstreben (5c, 7c) aufweisen, welche beim Einsetzen des Fluidbeutelhalters (100) in die Waagschale (200) nicht auf oberen Flächen oder Stirnseiten der Erhebung (201a, 203a) zum Liegen kommen.

11. Wiegevorrichtung nach einem der Ansprüche 7 bis 10, wobei die Stützarme (5, 7) Seitenstreben (5a, 5b, 7a, 7b) aufweisen, welche unter einem ersten Winkel zueinander stehen, wobei die Erhebungen (201a, 203a) Seitenflächen aufweisen, welche unter einem zweiten Winkel zueinander stehen, und wobei der erste Winkel und der zweite Winkel gleich oder im Wesentlichen gleich sind.

12. Wiegevorrichtung nach einem der Ansprüche 7 bis 11, wobei die Stützarme (5, 7) Querstreben (5c, 7c) aufweisen, welche beim Einsetzen des Fluidbeutelhalters (100) parallel oder im Wesentlichen parallel zu den oberen Flächen oder Stirnseiten der Erhebung (201a, 203a) angeordnet sind.

13. Wiegevorrichtung nach einem der Ansprüche 7 bis 12, wobei die Waagschale (200) eine Grundfläche (205) aufweist, insbesondere von welcher sich die Seitenflächen (201, 203) erstrecken, wobei die Grundfläche (205) länger ist als der Bodenabschnitt (1) oder als der Abstand von zwei bodenseitigen Abschnitten, vorgesehen jeweils auf einem der zwei Beutelhaltearmen (9, 11), voneinander.

14. Wiegevorrichtung nach einem der Ansprüche 7 bis 13, wobei die Waagschale (200) an ihren Längsseiten Seitenflächen (201, 203) aufweist, nicht jedoch an ihren Querseiten.

15. Medizinische Behandlungsvorrichtung, aufweisend wenigstens einen Fluidbeutelhalter (100) gemäß einem der Ansprüche 1 bis 5 oder wenigstens eine Wiegevorrichtung nach einem der Ansprüche 7 bis 14, ferner aufweisend wenigstens ein Halteblech (301, 303) zum Eingreifen in die Rückseite der Erhebung (201a, 203a) der Waagschale (200) der Wiegevorrichtung.

## Claims

1. A medical fluid bag rack (100) to be inserted into a scale pan (200) of a weighing apparatus and to hang up bags containing medical fluid, wherein the fluid bag rack (100), extending in a direction of its height (H),
in a direction of its length (L) perpendicular thereto, and perpendicular to the two directions mentioned above, in a direction of its width (B), respectively comprises:
- at least one base section (1);
- at least one bag rack arm (9, 11) extending from the base section (1), wherein the at least one bag rack arm (9, 11) comprises one or more hooks (13, 15) for hanging up one or more fluid bags;
or consists thereof,
**characterized in that** the fluid bag rack (100) further
- comprises at least two support arms (5, 7) extending from the base section (1),
wherein at least two of the support arms (5, 7) are connected, respectively, to the base section (1) at or on sides lying opposite to each other, or extend as one piece or integrally therefrom,
wherein at least one of the support arms (5, 7) comprises two lateral struts (5a, 5b, 7a, 7b) for supporting the fluid bag rack (100) by means of upper sections of the lateral struts (5a, 5b, 7a 7b) on upwardly extending lateral surfaces (201, 203) of the scale pan (200) while preventing rotation or tilting of the fluid bag rack (100) about an axis running in the direction of its width (B), which are spaced from one another by an open gap or an enclosed passage area or passage opening (5d, 7d).

2. The medical fluid bag rack (100) according to claim 1, wherein the base section (1) and the at least two support arms (5, 7) are made one piece, preferably from a common, first strip of material, in particular metal strip (21).

3. The medical fluid bag rack (100) according to anyone of the preceding claims, wherein the base section (1) and the at least one bag rack arm (9, 11) are made one piece, preferably from a common first strip of material, in particular metal strip (21).

4. The medical fluid bag rack (100) according to anyone of the preceding claims, wherein one of the support arms (5, 7) and the at least one bag rack arm (9, 11) are made one piece, preferably from a common first strip of material, in particular metal strip (21).

5. The medical fluid bag rack (100) according to anyone of the preceding claims, which comprises at least two bag rack arms (9, 11) connected to each other by means of a connecting section (17), preferably one piece.

6. A method for manufacturing a fluid bag rack (100) according to anyone of the preceding claims, including or consisting of the following steps:
- punching or laser cutting of one, two or more metal strips or blanks to produce a base section (1), at least two support arms (5, 7) extending from the base section (1), and at least one bag rack arm (9, 11), wherein the base section (1), the support arms (5, 7) and the bag rack arm (9, 11) are comprised by one or two metal strips (21, 23) ;
- bending and/or canting the sheet metal strip (s) (21, 23) ; and
- connecting the one metal strip (21) to itself or the two or more metal strips (21, 23) to each other so as to create a closed or circumferential structure, the step of the connection being or including a welding step.

7. A medical weighing apparatus comprising at least each:
- a fluid bag rack (100) according to anyone of claims 1 to 5;
- a weighing unit;
- a scale pan (200), connected with a weighing unit, wherein the scale pan (200) comprises at least one lateral surface (201, 203) or another section with at least one protrusion (201a, 203a), the protrusion (201a, 203a) being suitable and/or dimensioned to be received in the gap or passage opening (5d, 7d) of the support arm (5, 7) of the fluid bag rack (100).

8. The weighing apparatus according to claim 7, wherein the support arms (5, 7) comprise lateral struts (5a, 5b, 7a, 7b), which come to rest with at least one section of the lateral struts (5a, 5b, 7a, 7b) on lateral surfaces or end faces of the protrusion (201a, 203a) when the fluid bag rack (100) has been completely inserted into the scale pan (200).

9. The weighing apparatus according to claim 8, wherein the at least one section, in which the lateral struts (5a, 5b, 7a, 7b) come into contact with at least one portion of the lateral struts (5a, 5b, 7a, 7b) on the lateral surfaces or end faces of the protrusion (201a, 203a), is part of that end section (5a', 5b', 7a', 7b') of the lateral struts (5a, 5b, 7a, 7b) facing away from the base section (1).

10. The weighing apparatus according to anyone of the claims 7 to 9, wherein the support arms (5, 7) comprise cross struts (5c, 7c) which do not come to rest on upper surfaces or end faces of the protrusion (201a, 203a) when the fluid bag rack (100) has been inserted into the scale pan (200).

11. The weighing apparatus according to anyone of the claims 7 to 10, wherein the support arms (5, 7) comprise lateral struts (5a, 5b, 7a, 7b), which are at a first angle to each other, wherein the protrusions (201a, 203a) comprise lateral surfaces which are at a second angle to each other, and wherein the first angle and the second angle are equal or practically equal.

12. The weighing apparatus according to anyone of the claims 7 to 11, wherein the support arms (5, 7) comprise cross struts (5c, 7c) which are arranged parallel or practically parallel to the upper surfaces or end faces of the protrusion (201a, 203a) when the fluid bag rack (100) has been inserted.

13. The weighing apparatus according to anyone of the claims 7 to 12, wherein the scale pan (200) comprises a base surface (205), especially from which the lateral surfaces (201, 203) extend, the base surface (205) being longer than the base section (1), or than the distance between two base-side sections, each provided relative to one another on one of the two bag rack arms (9, 11).

14. The weighing apparatus according to anyone of the claims 7 to 13, wherein the scale pan (200) comprises lateral surfaces (201, 203) on its longitudinal sides, but not on its transverse sides.

15. A medical treatment apparatus comprising at least a fluid bag rack (100) according to anyone of the claims 1 to 5 or at least a weighing apparatus according to anyone of the claims 7 to 14, further comprising at least one holding plate (301, 303) for intervening in the rear side of the protrusion (201a, 203a) of the scale pan (200) of the weighing apparatus.

## Revendications

1. Un porte-poche de fluide médical (100) destiné à être inséré dans un plateau de pesée (200) d'un appareil de pesée et permettant de suspendre des poches contenant du fluide médical, où le porte-poche de fluide (100), qui s'étend en direction de sa hauteur (H), en direction de sa longueur (L) de façon perpendiculaire à celle-ci, et de façon perpendiculaire aux deux directions mentionnées ci-dessus, en direction de sa largeur (B), respectivement, comprend:
- au moins une section de base (1);
- au moins un bras porte-poche (9, 11) qui s'étend de la section de base (1), où au moins un bras porte-poche (9, 11) comprend un ou plusieurs crochets (13, 15) pour suspendre une ou plusieurs poches de fluide;
ou est constitué de ceux-ci,
**caractérisé en ce que** le porte-poche de fluide (100)
- comprend en outre au moins deux bras de support (5, 7) qui s'étendent depuis la section de base (1),
où au moins deux des bras de support (5, 7) situés sur des côtés respectivement opposés de la section de base (1) sont reliés à celle-ci, ou en sortent d'un seul tenant ou intégralement,
où au moins un des bras de support (5, 7) comprend deux entretoises latérales (5a, 5b, 7a, 7b) destinées à soutenir le porte-poche de fluide (100) au moyen de parties supérieures des entretoises latérales (5a, 5b, 7a 7b) sur des surfaces latérales (201, 203) du plateau de pesée (200) s'étendant vers le haut, tout en empêchant la rotation ou le basculement du porte-poche de fluide (100) autour d'un axe en direction de sa largeur (B), lesquelles sont espacées les unes des autres par un interstice ouvert, une surface de passage enclavée ou une ouverture de passage (5d, 7d) .

2. Le porte-poche de fluide médical (100) selon la première revendication, où la section de base (1) et au moins les deux bras de support (5, 7) sont fabriqués d'un seul tenant, de préférence à partir d'une première bande de matériau commune, notamment d'une bande de tôle (21).

3. Le porte-poche de fluide médical (100) selon l'une quelconque des revendications précédentes, où la section de base (1) et au moins un bras porte-poche (9, 11) sont fabriqués d'un seul tenant, de préférence à partir d'une première bande de matériau commune, notamment d'une bande de tôle (21).

4. Le porte-poche de fluide médical (100) selon l'une quelconque des revendications précédentes, où un des bras de support (5, 7) et au moins un bras porte-poche (9, 11) sont fabriqués d'un seul tenant, de préférence à partir d'une première bande de matériau commune, notamment d'une bande de tôle (21).

5. Le porte-poche de fluide médical (100) selon l'une quelconque des revendications précédentes, qui comprend au moins deux bras porte-poche (9, 11) reliés l'un à l'autre au moyen d'une section de raccordement (17), de préférence d'un seul tenant.

6. Un procédé de fabrication d'un porte-poche de fluide (100) selon l'une quelconque des revendications précédentes, comprenant, ou consistant en, les étapes suivantes:
- le poinçonnage ou la découpe au laser d'une, de deux, ou de plusieurs bandes de tôle ou d'ébauches pour créer une section de base (1), au moins deux bras de support (5, 7) s'étendant de la section de base (1), et au moins un bras porte-poche (9, 11), où la section de base (1), les bras de support (5, 7) ainsi que le bras porte-poche (9, 11) se situent sur une ou deux bandes de tôle (21, 23);
- le pliage et/ou le chanfreinage de la ou des bandes(s) de tôle (21, 23); et
- la connexion d'une bande de tôle (21) avec elle-même ou de deux ou de plusieurs bandes de tôle (21, 23) entre elles, de manière à créer une structure fermée ou circonférentielle, l'étape de connexion étant, ou comprenant, une étape de soudage.

7. Un appareil de pesée médical comprenant au moins:
- un porte-poche de fluide (100) selon l'une quelconque des revendications 1 à 5;
- un dispositif de pesée;
- un plateau de pesée (200), relié à un dispositif de pesée, où le plateau de pesée (200) comprend au moins une surface latérale (201, 203), ou une autre partie, dotée d'au moins une saillie (201a, 203a), la saillie (201a, 203a) étant adaptée et/ou dimensionnée pour être reçue dans l'interstice ou dans l'ouverture de passage (5d, 7d) du bras de support (5, 7) du porte-poche de fluide (100).

8. L'appareil de pesée selon la revendication 7, où les bras de support (5, 7) comprennent des entretoises latérales (5a, 5b, 7a, 7b), qui viennent prendre appui avec au moins une section des entretoises latérales (5a, 5b, 7a, 7b) sur des surfaces latérales ou des faces frontales de la saillie (201a, 203a) lors de l'insertion complète du porte-poche de fluide (100) dans le plateau de pesée (200).

9. L'appareil de pesée selon la revendication 8, où au moins la section dans laquelle les entretoises latérales (5a, 5b, 7a, 7b) entrent en contact avec au moins une section des entretoises latérales (5a, 5b, 7a, 7b) au niveau des surfaces latérales ou des faces frontales de la saillie (201a, 203a), fait partie de cette extrémité (5a', 5b', 7a', 7b') des entretoises latérales (5a, 5b, 7a, 7b) opposée à la section de base (1).

10. L'appareil de pesée selon l'une quelconque des revendications 7 à 9, où les bras de support (5, 7) comportent des croisillons (5c, 7c), qui ne viennent pas prendre appui sur les surfaces supérieures ou les faces frontales de la saillie (201a, 203a) lors de l'insertion du porte-poche de fluide (100) dans le plateau de pesée (200).

11. L'appareil de pesée selon l'une quelconque des revendications 7 à 10, où les bras de support (5, 7) comportent des entretoises latérales (5a, 5b, 7a, 7b) qui sont à un premier angle les unes par rapport aux autres, les saillies (201a, 203a) comportant des surfaces latérales qui sont à un second angle les unes par rapport aux autres, et où le premier angle et le second angle sont égaux ou pratiquement égaux.

12. L'appareil de pesée selon l'une quelconque des revendications 7 à 11, où les bras de support (5, 7) comprennent des croisillons (5c, 7c), qui sont agencés de façon parallèle ou pratiquement parallèle aux surfaces supérieures ou aux faces frontales de la saillie (201a, 203a) lors de l'insertion du porte-poche de fluide (100).

13. L'appareil de pesée selon l'une quelconque des revendications 7 à 12, où le plateau de pesée (200) comprend une surface de base (205), notamment à partir de laquelle s'étendent les surfaces latérales (201, 203), la surface de base (205) étant plus longue que la section de base (1) ou que la distance entre deux sections latérales de base, chacune prévue sur l'un des deux bras porte-poche (9, 11) l'une par rapport à l'autre.

14. L'appareil de pesée selon l'une quelconque des revendications 7 à 13, où le plateau de pesée (200) comprend des surfaces latérales (201, 203) sur ses côtés longitudinaux, mais pas sur ses côtés transversaux.

15. Un appareil de traitement médical comprenant au moins un porte-poche de fluide (100) selon l'une quelconque des revendications 1 à 5 ou au moins un appareil de pesée selon l'une quelconque des revendications 7 à 14, comprenant en outre au moins une tôle de support (301, 303) destinée à intervenir à l'arrière de la saillie (201a, 203a) du plateau de pesée (200) de l'appareil de pesée.
